(19) Europäisches Patentamt
European Patent Office
Office européen des brevets

(11) Veröffentlichungsnummer: **0 025 997**
**B1**

# (12) EUROPÄISCHE PATENTSCHRIFT

(45) Veröffentlichungstag der Patentschrift: **13.04.83**

(51) Int. Cl.³: **C 07 C 87/20,** C 07 C 85/08, C 07 C 85/24

(21) Anmeldenummer: **80105667.2**

(22) Anmeldetag: **20.09.80**

(54) **N,N'-Dialkylaminoalkylethylendiamine und Verfahren zu deren Herstellung.**

(30) Priorität: **25.09.79 DE 2938710**

(43) Veröffentlichungstag der Anmeldung: **01.04.81 Patentblatt 81/13**

(45) Bekanntmachung des Hinweises auf die Patenterteilung: **13.04.83 Patentblatt 83/15**

(84) Benannte Vertragsstaaten: **AT BE CH DE FR GB IT LI NL SE**

(56) Entgegenhaltungen:
**DE-A-2 738 538**
**DE-A-2 833 170**
**DE-B-2 641 836**
**CHEMISCHE BERICHTE, Band 109, 1976 H. TOM DIECK et al. »Metallierung einer nichtaktivierten Alkyl-Gruppe im Nickelkomplex« Seiten 1657 bis 1664**
**K. Lindner Tenside-Textilhilfsmittel-Waschrohstoffe, Bd. I (1964) S. 425**

(73) Patentinhaber: **HOECHST AKTIENGESELLSCHAFT, Postfach 80 03 20, D-6230 Frankfurt/Main 80 (DE)**

(72) Erfinder: **Diery, Helmut, Dr., Theresenstrasse 45, D-6233 Kelkheim (Taunus) (DE)**
Erfinder: **Wagemann, Wolfgang, Dr., Beektwiete 2a, D-2071 Tremsbüttel (DE)**

**EP 0 025 997 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# N,N'-Dialkylaminoalkylethylendiamine und Verfahren zu deren Herstellung

Gegenstand der Erfindung sind N,N'-Dialkylaminoalkylethylendiamine der Formel 1

$$R_1-NH-(CH_2)_m-NH-(CH_2)_2-NH-(CH_2)_n-NH-R_2 \quad (1)$$

worin $R_1$ und $R_2$ gleich oder verschieden sind und Alkyl, 2-Hydroxialkyl oder Alkenyl mit jeweils 8 bis 30 C-Atomen oder $C_8-C_{24}$-Alkoxi-propyl und m und n 2 oder 3 bedeuten.

Bevorzugt sind solche Verbindungen der Formel 1, worin $R_1$ und $R_2$ identisch sind und Alkyl, 2-Hydroxialkyl oder Alkenyl mit jeweils 8 bis 18 C-Atomen und $m = n = 3$ bedeuten.

Ebenfalls Gegenstand der vorliegenden Erfindung ist ein Verfahren zur Herstellung der Verbindungen der Formel 1, indem man Fettalkylalkylendiamine der Formel 2

$$R_1-NH-(CH_2)_m-NH_2$$

bzw. $\quad (2)$

$$R_2-NH-(CH_2)_n-NH_2$$

mit Glyoxal zu Bisaldiminen der Formel 3 umsetzt

$$R_1-NH-(CH_2)_{m(n)}-N=CH-CH=N-(CH_2)_{n(m)}-NH-R_2 \quad (3)$$

und diese Schiffschen Basen anschließend katalytisch hydriert.

Als Alkylalkylendiamine der Formel 2 kann man zum Beispiel Cocosalkylpropylendiamin, Stearylpropylendiamin oder Talgfettpropylendiamin einsetzen. Erfindungsgemäß können auch die entsprechenden Ethylendiamine wie z. B. N-Dodecylethylendiamin oder N-Cetylethylendiamin verwendet werden oder auch Etheralkylendiamine wie z. B. Lauryloxipropylpropylendiamin.

In einer besonders bevorzugten Ausführungsform der Erfindung wählt man in der Formel 1 $R_1$ gleich $R_2$ und $m = n = 3$. Vorteilhaft geeignete und in technischen Mengen verfügbare Ausgangsprodukte hierzu sind z. B. Fettalkylpropylendiamine auf Basis natürlicher Fette und Öle wie Talg oder Cocosöl. Im Rahmen der vorliegenden Erfindung können selbstverständlich auch Diamine mit technisch nicht verfügbarer Kettenverteilung oder kettenreine Verbindungen verwendet werden. Diese Substanzen stellt man in bekannter Weise z. B. durch Anlagerung von 1 Mol Acrylnitril an primäre Fettamine und anschließende katalytische Hydrierung der Propionitrile her. Die entsprechenden Alkoxipropylpropylendiamine erhält man durch Anlagerung von Acrylnitril an Fettalkohole, katalytische Hydrierung der Propionitrile, Anlagerung eines weiteren Moleküls Acrylnitril an die resultierenden Alkoxipropylamine und erneute katalytische Hydrierung. Die Alkylethylendiamine lassen sich beispielsweise durch Umsetzung von einem Mol Alkylhalogenid mit Ethylendiamin herstellen.

Die Kondensation der Diamine der Formel 2 mit Glyoxal wird bei Temperaturen unter 50°C, vorzugsweise bei Raumtemperatur durchgeführt, indem man zu einer Lösung des Diamins in einem bezüglich der Reaktion indifferenten Lösungsmittel, vorzugsweise in einem Alkohol wie Methanol, Isopropanol oder Isobutanol, eine 40%ige handelsübliche wäßrige Glyoxal-Lösung gibt. Das Molverhältnis von Amin zu Glyoxal liegt zwischen 2 : 1 und 2 : 1,2. Die Bildung der Bisaldimine findet augenblicklich statt. Insbesondere bei Verwendung von Methanol als Lösungsmittel sind die Produkte schwerlöslich und setzen sich in flüssiger, pastöser oder fester Form ab. Sie können durch Phasentrennung bzw. Filtration leicht abgetrennt und nach Trocknung als Reinsubstanzen isoliert werden.

Vorzugsweise wird man jedoch die bei der Kondensation anfallenden Suspensionen oder Emulsionen der Bisaldimine der Formel 3 ohne Isolierung direkt der katalytischen Hydrierung unterwerfen. Man kann erfindungsgemäß natürlich auch 2 Mol verschiedener N-Alkylpropylendiamine, N-Alkylethylendiamine oder N-Alkoxipropylpropylendiamine oder deren Gemische mit Glyoxal umsetzen, wobei statistische Mischungen verschiedener Schiffscher Basen erhalten werden.

Die katalytische Hydrierung führt man zweckmäßigerweise bei Temperaturen unter 100°C, vorzugsweise bei 40 bis 80°C durch. Als Katalysatoren eignen sich Edelmetalle wie Platin, Palladium oder Rhodium. Aus wirtschaftlichen Erwägungen wird man jedoch vorzugsweise Metallkatalysatoren wie Nickel oder Cobalt verwenden. Insbesondere eignet sich Raney-Nickel. Man arbeitet erfindungsgemäß mit Wasserstoff-Arbeitsdrücken von 50 bis 150 bar, insbesondere bei 80 bis 120 bar.

Die Reinisolierung der erfindungsgemäßen Produkte der Formel 1 geschieht durch Abfiltrieren vom Hydrierungskatalysator und Abdampfen des Lösungsmittels. Die resultierenden Verbindungen der Formel 1 liegen gemäß Analyse des Aminstickstoffs in 80- bis 90%iger Reinheit vor und können für die meisten Verwendungszwecke in dieser Form direkt weiterverwendet werden. Völlig reine Verbindungen können durch Umkristallisation gewonnen werden.

Die erfindungsgemäßen N,N'-Dialkylalkylenethylendiamine der Formel 1 eignen sich als Ausgangsprodukte zur Herstellung einer ganzen Reihe neuartiger oberflächenaktiver Verbindungen.

## Beispiel 1

900 g (2,5 Mol) technisches Talgfettpropylendiamin mit einer Alkylkettenverteilung von etwa 1% $C_{12}$, 3% $C_{14}$, 31% $C_{16}$, 35% $C_{18}$ und 30% $C_{18}$ ungesättigt werden in 2500 ml Methanol gelöst. Bei Raumtemperatur tropft man innerhalb von 30 Minuten 190 g (1,3 Mol) 40%ige Glyoxal-Lösung hinzu und rührt anschließend 2 Stunden bei Raumtemperatur nach. Das rohe Reaktionsgemisch wird in einen Edelstahl-Autoklav überführt, mit 16 g Raney-Nickel versetzt und unter Rühren bis zum Ende der Wasserstoffaufnahme bei 60°C und 100 bar Wasserstoffdruck hydriert. Anschließend filtriert man über eine beheizte Druckfilternutsche vom Katalysator ab und verdampft das Methanol im Vakuum. Man erhält 900 g hellbeigefarbenes wachsartiges Produkt der Formel

$$R-NH-(CH_2)_3-NH-(CH_2)_2-NH-(CH_2)_3-NH-R$$

R = Talgfettalkyl

mit einem Gehalt an 6% sekundärem Aminstickstoff und 0,8% tertiärem Aminstickstoff.

## Beispiel 2

133,5 g (0,7 Mol) Octylpropylendiamin werden in 650 ml Methanol gelöst. Bei Raumtemperatur tropft man innerhalb von 15 Minuten 54,8 g (0,38 Mol) 40%ige wäßrige Glyoxal-Lösung hinzu und rührt anschließend 2 Stunden bei Raumtemperatur nach. Das rohe Reaktionsgemisch wird in einen Edelstahl-Autoklav überführt und nach Zugabe von 5 g Raney-Nickel bei 60°C und 100 bar Wasserstoffdruck bis zum Ende der Wasserstoffaufnahme hydriert. Nach Filtration über eine Druckfilternutsche und Verdampfen des Lösungsmittels resultieren 135 g hellbraunes flüssiges Produkt der Formel

$$C_8H_{17}NH-(CH_2)_3-NH-(CH_2)_2-NH-(CH_2)_3-NHC_8H_{17}$$

mit einem Gehalt von 9,9% sekundärem Aminstickstoff und 1,3% tertiärem Aminstickstoff.

## Beispiel 3

1408 g (5,5 Mol) eines destillierten Laurylpropylendiamins mit einer Alkylkettenverteilung von etwa 75% $C_{12}$ und 25% $C_{14}$ werden in 5,5 Litern Methanol gelöst. Bei Raumtemperatur tropft man innerhalb von 30 Minuten 433 g (2,98 Mol) 40%ige wäßrige Glyoxal-Lösung hinzu und rührt anschließend 2 Stunden bei Raumtemperatur nach. Das rohe Reaktionsgemisch wird in einen Edelstahl-Autoklav überführt und nach Zugabe von 38 g Raney-Nickel bei 60°C einem Wasserstoffdruck von 90 bar bis zum Ende der Wasserstoffaufnahme hydriert. Nach Filtration über eine Druckfilternutsche und Verdampfen des Lösungsmittels erhält man 1400 g hellbeigefarbenes wachsartiges Produkt der Formel

$$R-NH-(CH_2)_3-NH-(CH_2)_2-NH-(CH_2)_3-NH-R$$

R = 75% $C_{12}$-Alkyl und 25% $C_{14}$-Alkyl

mit einem Gehalt von 8,05% sekundärem und 0,95% tertiärem Aminstickstoff.

## Beispiel 4

560 g (2 Mol) eines destillierten Cocospropylendiamins mit einer Alkylkettenverteilung von etwa 7% $C_8$, 6% $C_{10}$, 51% $C_{12}$, 19% $C_{14}$, 8% $C_{16}$ und 9% $C_{18}$ werden in 1700 ml Methanol gelöst. Bei Raumtemperatur tropft man innerhalb von 30 Minuten 157,2 g (1,08 Mol) 40%ige wäßrige Glyoxal-Lösung hinzu und rührt anschließend 2 Stunden bei Raumtemperatur nach. Das rohe Reaktionsgemisch wird in einen Edelstahl-Autoklav überführt und nach Zugabe von 12 g Raney-Nickel drei Stunden lang bei 60°C und einem Wasserstoffdruck von 100 bar hydriert. Nach Aufarbeitung wie in den vorhergehenden Beispielen beschrieben, resultieren 550 g gelbes flüssiges Produkt der Formel

$$R-NH-(CH_2)_3-NH-(CH_2)_2-NH-(CH_2)_3-NH-R$$

R = Cocosalkyl mit einem Gehalt an 7,75% sekundärem und 0,8% tertiärem Aminstickstoff.

## Beispiel 5

390 g (1,5 Mol) eines destillierten Isotridecylpropylendiamins werden in 1300 ml Methanol gelöst. Bei Raumtemperatur tropft man innerhalb von 40 Minuten 116 g (0,8 Mol) 40%ige wäßrige Glyoxal-Lösung hinzu und rührt anschließend 2 Stunden bei Raumtemperatur nach. Das rohe Reaktionsgemisch wird in einen Edelstahl-Autoklav überführt und nach Zugabe von 10 g Raney-Nickel vier Stunden bei 70° C und einem Wasserstoffdruck von 105 bar hydriert. Nach Aufarbeitung wie vorbeschrieben resultieren 392 g orangefarbenes viskoses flüssiges Produkt der Formel

$$R-NH-(CH_2)_3-NH-(CH_2)_2-NH-(CH_2)_3-NH-R$$

R = Isotridecyl mit einem Gehalt an 9,1% sekundärem und 0,9% tertiärem Aminstickstoff.

## Beispiel 6

800 g (2,5 Mol) eines Alkoxipropylpropylendiamins, dessen Alkoxirest eine Kettenverteilung von $C_{12}$ bis $C_{15}$ aufweist und zu etwa 80% linear, zu etwa 20% verzweigt ist, werden in 2500 ml Methanol gelöst. Man tropft innerhalb 35 Minuten bei Raumtemperatur 190 g (1,3 Mol) 40%ige Glyoxallösung hinzu und rührt 2 Stunden bei Raumtemperatur nach. Das rohe Reaktionsgemisch wird in einem Edelstahl-Autoklav nach Zugabe von 15 g Raney-Nickel 3 1/2 Stunden bei 60° C und 95 bar Wasserstoffdruck hydriert. Nach der Aufarbeitung (s. o.) resultieren 790 g gelbes Öl der Verbindung der Formel

$$R-NH-(CH_2)_3-NH-(CH_2)_2-NH-(CH_2)_3-NH-R$$

R = $C_{12}/C_{15}$-Alkoxypropyl mit einem Gehalt an 7,1% sekundärem und 0,8% tertiärem Aminstickstoff.

## Patentansprüche für Vertragsstaaten: BE, CH, LI, DE, FR, GB, IT, NL, SE

1. N,N'-Dialkylaminoalkylenethylendiamine der Formel 1

$$R_1-NH-(CH_2)_m-NH-(CH_2)_2-NH-(CH_2)_n-NH-R_2 \qquad (1)$$

worin $R_1$ und $R_2$ gleich oder verschieden sind und Alkyl, 2-Hydroxialkyl oder Alkenyl mit jeweils 8 bis 30 C-Atomen oder $C_8-C_{24}$-Alkoxi-propyl und m und n 2 oder 3 bedeuten.

2. Verbindungen der Formel 1 nach Anspruch 1, worin $R_1$ und $R_2$ identisch sind und Alkyl, 2-Hydroxialkyl oder Alkenyl jeweils mit 8 bis 18 C-Atomen und m = n = 3 bedeuten.

3. Verfahren zur Herstellung der Verbindungen der Formel 1 nach Anspruch 1, dadurch gekennzeichnet, daß man zunächst Alkylalkylendiamine der Formel 2

$$R_1-NH-(CH_2)_m-NH_2$$

bzw. (2)

$$R_2-NH-(CH_2)_n-NH_2$$

mit Glyoxal zu Bisaldiminen der Formel 3

$$R_1-NH-(CH_2)_{m(n)}-N=CH-CH=N-(CH_2)_{n(m)}-NH-R_2 \qquad (3)$$

kondensiert und diese anschließend katalytisch zu der Verbindung der Formel 1 hydriert.

## Patentansprüche für Vertragsstaat: AT

1. Verfahren zur Herstellung von N,N'-Dialkylaminoalkylenethylendiaminen der Formel 1

$$R_1-NH-(CH_2)_m-NH-(CH_2)_2-NH-(CH_2)_n-NH-R_2(1)$$

worin $R_1$ und $R_2$ gleich oder verschieden sind und Alkyl, 2-Hydroxialkyl oder Alkenyl mit jeweils 8 bis 30 C-Atomen oder $C_8-C_{24}$-Alkoxi-propyl und m und n 2 oder 3 bedeuten, dadurch gekennzeichnet, daß man zunächst Alkylalkylendiamine der Formel 2

$$R_1-NH-(CH_2)_m-NH_2$$

bzw. (2)

$$R_2-NH-(CH_2)_n-NH_2$$

mit Glyoxal zu Bisaldiminen der Formel 3

$$R_1-NH-(CH_2)_{m(n)}-N=CH-CH=N-(CH_2)_{n(m)}-NH-R_2 \quad (3)$$

kondensiert und diese anschließend katalytisch zu der Verbindung der Formel 1 hydriert.

2. Verfahren nach Anspruch 1, dadurch gekennzeichnet, daß man solche N,N'-Dialkylaminoalkyl-ethylendiamine der Formel 1 herstellt, worin $R_1$ und $R_2$ identisch sind und Alkyl, 2-Hydroxialkyl oder Alkenyl jeweils mit 8 bis 18 C-Atomen und $m=n=3$ bedeuten.

**Claims for the Contracting states: BE, CH, DE, FR, GB, IT, LI, NL, SE**

1. N,N'-dialkylaminoalkyl-ethylenediamines of the formula 1

$$R_1-NH-(CH_2)_m-NH-(CH_2)_2-NH-(CH_2)_n-NH-R_2 \quad (1)$$

wherein $R_1$ and $R_2$ are identical or different and mean alkyl, 2-hydroxyalkyl or alkenyl with from 8 to 30 carbon atoms each or $C_8-C_{24}$-alkoxy-propyl and m and n are 2 or 3.

2. Compounds of the formula 1 according to claim 1, wherein $R_1$ and $R_2$ are identical and mean alkyl, 2-hydroxyalkyl or alkenyl with from 8 to 18 carbon atoms each and $m=n=3$.

3. Process for the manufacture of compounds of the formula 1 according to claim 1, which comprises first condensing alkyl-alkylenediamines of the formula 2

$$R_1-NH-(CH_2)_m-NH_2$$

or (2)

$$R_2-NH-(CH_2)_n-NH_2$$

with glyoxal to give bisaldimines of the formula 3

$$R_1-NH-(CH_2)_{m(n)}-N=CH-CH=N-(CH_2)_{n(m)}-NH-R_2 \quad (3)$$

and subsequently hydrogenating same catalytically to give the compound of the formula 1.

**Claims for the Contracting state: AT**

1. Process for the manufacture of N,N'-dialkylaminoalkyl-ethylenediamines of the formula 1

$$R_1-NH-(CH_2)_m-NH-(CH_2)_2-NH-(CH_2)_n-NH-R_2 \quad (1)$$

wherein $R_1$ and $R_2$ are identical or different and mean alkyl, 2-hydroxyalkyl or alkenyl with from 8 to 30 carbon atoms each or $C_8-C_{24}$-alkoxy-propyl and m and n are 2 or 3, which comprises first condensing alkyl-alkylenediamines of the formula 2

$$R_1-NH-(CH_2)_m-NH_2$$

or (2)

$$R_2-NH-(CH_2)_n-NH_2$$

with glyoxal to give bisaldimines of the formula 3

$$R_1-NH-(CH_2)_{m(n)}-N=CH-CH=N-(CH_2)_{n(m)}-NH-R_2 \quad (3)$$

and subsequently hydrogenating same catalytically to give the compound of the formula 1.

2. Process as claimed in claim 1 which comprises preparing N,N'-dialkylaminoalkyl-ethylenediamines of the formula 1 wherein $R_1$ and $R_2$ are identical and mean alkyl, 2-hydroxyalkyl or alkenyl with from 8 to 18 carbon atoms each and $m=n=3$.

**Revendications pour les Etats contractants: BE, CH, DE, FR, GB, IT, LI, NL, SE**

1. N,N'-Bis-(alkylaminoalkyl)-éthylène-diamines répondant à la formule 1

$$R_1-NH-(CH_2)_m-NH-(CH_2)_2-NH-(CH_2)_n-NH-R_2 \quad (1)$$

dans laquelle $R_1$ et $R_2$, qui sont identiques ou différents, représentent des radicaux alkyles, hydroxy-2 alkyles ou alcényles contenant chacun de 8 à 30 atomes de carbone ou des radicaux alcoxy-propyles dont la partie alcoxy contient de 8 à 24 atomes de carbone, et m et n sont égaux chacun à 2 ou à 3.

2. Composés de formule 1 selon la revendication 1, dans lesquels $R_1$ et $R_2$ sont identiques et représentent chacun un radical alkyle, hydroxy-2 alkyle ou alcényle contenant de 8 à 18 atomes de carbone, et m et n sont égaux chacun à 3.

3. Procédé de préparation des composés de formule 1 selon la revendication 1, procédé caractérisé en ce qu'on condense d'abord des alkyl-alkylène-diamines de formules 2

$$\begin{matrix} R_1-NH-(CH_2)_m-NH_2 \\ R_2-NH-(CH_2)_n-NH_2 \end{matrix} \quad (2)$$

avec le glyoxal de manière à obtenir des bis-aldimines de formule 3

$$R_1-NH-(CH_2)_{m(n)}-N=CH-CH=N-(CH_2)_{n(m)}-NH-R_2 \quad (3)$$

puis on hydrogène catalytiquement ces dernières pour les convertir en composés de formule 1.

**Revendications pour l'Etat contractant: AT**

1. Procédé de préparation de N,N'-bis-(alkylaminoalkyl)-éthylène-diamines répondant à la formule 1

$$R_1-NH-(CH_2)_m-NH-(CH_2)_2-NH-(CH_2)_n-NH-R_2 \quad (1)$$

dans laquelle $R_1$ et $R_2$, qui sont identiques ou différents, représentent des radicaux alkyles, hydroxy-2 alkyles ou alcényles contenant chacun de 8 à 30 atomes de carbone ou des radicaux alcoxy-propyles dont la partie alcoxy contient de 8 à 24 atomes de carbone, et m et n sont égaux chacun à 2 ou à 3, procédé caractérisé en ce qu'on condense d'abord des alkyl-alkylène-diamines de formules 2

$$\begin{matrix} R_1-NH-(CH_2)_m-NH_2 \\ R_2-NH-(CH_2)_n-NH_2 \end{matrix} \quad (2)$$

avec le glyoxal de manière à obtenir des bis-aldimines de formule 3

$$R_1-NH-(CH_2)_{m(n)}-N=CH-CH=N-(CH_2)_{n(m)}-NH-R_2 \quad (3)$$

puis on hydrogène catalytiquement ces dernières pour les convertir en composés de formule 1.

2. Procédé selon la revendication 1, caractérisé en ce qu'on prépare des N,N'-bis-(alkylaminoalkyl)-éthylène-diamines de formule 1 dans lesquelles $R_1$ et $R_2$ sont identiques et représentent chacun un radical alkyle, hydroxy-2 alkyle ou alcényle contenant de 8 à 18 atomes de carbone, et m et n sont égaux chacun à 3.